# EUROPEAN PATENT APPLICATION

(11) **EP 2 100 613 A1**
(43) Date of publication of application: **16.09.2009**
(21) Application number: 08152718.6
(22) Date of filing: 13.03.2008
(51) Int. Cl.: A61K 36/185, A61P 25/24, A23G 1/54, A23G 3/54, A21D 13/00

(54) **Chocolate and biscuit combination for uplifting the mood**

(71) Applicant: Nestec S.A., 1800 Vevey (CH)
(72) Inventor: Rezzi, Serge, 1066, EPALINGES (CH); Ramadan, Ziad, MANCHESTER, MO 63021 (US); Kochhar, Sunil, 1073, SAVIGNY (CH); Martin, François-Pierre, 01170, CESSY (FR); Fay, Laurent-Bernard, 74500, EVIAN (FR); Mollet, Beat, CH 1012 Lausanne (CH)

(57) **Abstract**

A method of uplifting the mood of a human subject comprises administering a combination of chocolate and biscuit for consumption by the subject. A composition comprising a combination of chocolate and biscuit is also provided for use in for such a method for example for the treatment of anxiety or stress.

## Description

### Field of the invention

The present invention relates to the use of a combination of chocolate and biscuit, in particular milk chocolate and wafer for uplifting the mood of a human subject.

### Background of the invention

Chocolate is one of the best-loved foods everywhere in the world. The attractive tastes and textures of chocolate and chocolate products delight the senses and make them well-known comfort foods. Studies have indeed demonstrated that primarily taste, texture, and aroma play an important role in the feeling of satisfaction experienced after chocolate consumption (1). Aside from the initial sensory benefits, consumers may choose chocolate or a chocolate containing product for a whole range of reasons: its satiating qualities, its caloric value, the post-prandial feeling it provides or the price/quality ratio.

Several publications report the relationship between anxiety and mood. Stress and mood are also tightly connected (2, 3). Furthermore, some publications have established a relation between mood and energy level (4, 5). In terms of the affective experience, chocolate has traditionally been associated with changes in emotional experience. Eating chocolate is associated with positive emotions such as feelings of pleasure, joy and success. Alternatively, experiencing negative affective states such as sorrow, depression and stress can lead to increased chocolate consumption (6, 7). Only a few scientific studies have addressed chocolate's hedonic appeal and mental effects (8, 9), although evidence is sparse and not very solid, there have been suggestions that chocolate's mood elevating properties reflect 'drug-like' constituents including, phenylethylamine, methylxanthines (caffeine, theobromine), and magnesium. Whilst the concentrations of these substances in chocolate are normally too low to induce a "pharmacological response", it was previously reported that the combination of caffeine and theobromine in a normal portion of 50 g of chocolate exhibit psychopharmacological activity (10). As most palatable foods stimulate endorphin release in the brain, this may be a potential mechanism to account for the elevation of mood (11). Carbohydrate-rich foods are known for providing important metabolic fuel for physical performance, but their effects on cognitive performance are not very clear (12, 13). Although increases in blood glucose levels following food intake affect cognitive performance in the postprandial period, different types of carbohydrates also have different types of effects (14).

In general, nutrition studies focus on the effects of individual ingredients of a product. The present invention is based on a study, the main focus of which was to assess the effects of chocolate and biscuit consumption on state anxiety, state energy, state emotion and cognition in high and low trait anxiety subjects.

### Summary of the invention

The present invention provides a method of uplifting the mood of a human subject which comprises administering to a human subject in need thereof a combination of chocolate and biscuit. In the context of the invention an uplifted mood may comprise a decreased anxiety level and/or a decreased stress level and/or an increased perceived energy level and/or a more positive emotional state.

The present invention also provides a combination of chocolate and biscuit for use in a method of uplifting the mood of a human subject by administering to a human subject in need thereof a combination of chocolate and biscuit. For example the composition may be for the treatment of anxiety or stress. The composition may be particularly suitable for uplifting the mood of or for the treatment of anxiety or stress in a high trait anxiety individual.

### Brief description of the drawings

Figure 1 shows the mean (±seem) state anxiety score per treatment for cream cracker, KitKat® and Noir Intense reported by subjects in the high trait anxiety group. A higher reported score is associated with a lower experienced stress.
Figure 2 shows the mean (±sem) of perceived energy levels score per treatment for cream cracker, KitKat® and Noir Intense reported by high trait anxiety subjects. A higher reported score is associated with a higher energy state.
Figure 3 shows the mean (± sem) of perceived emotion score per treatment for cream cracker, KitKat® and Noir Intense reported by the subjects in the high trait anxiety group. A higher reported score is associated with a better emotional state.

### Detailed description of the invention

The invention deals with the use of the combination of chocolate with biscuit to uplift mood, for example to improve stress, energy and emotional states in the general population. The term 'uplifting' may be defined in many ways. Herein we use a multifacetted definition of the concept 'mood uplifting' comprising a more positive affective state (emotion), an increased subjective energy level and decreased stress and anxiety level.

The invention is based on scientific results obtained from a clinical trial in which anxiety, energy and emotional states were measured in high and low trait anxiety human subjects after consumption of KitKat®, dark chocolate and an isocaloric food (cream cracker with cheese). The results showed that treatments have significant effects in high trait anxiety individuals. In particular, consuming a KitKat® has a positive effect on subjects, who become less stressed (are less anxious), feel more energetic, and, to a lesser extent, become happier. This effect contrasts with consuming the isocaloric substance which was associated with higher stress and with consuming dark chocolate which had no effect on state anxiety, energy levels or emotional state as a function of trait anxiety and time. All effects were found for short-term consumption and no long-term effects over the design were observed. In addition, none of the treatments were found as statistically significant for improving cognitive performance.

The present invention thus provides a method of uplifting the mood of a human subject which comprises administering a combination of chocolate and biscuit for consumption by the human subject. The invention also provides a composition comprising a combination of chocolate and biscuit for use in such a method. In the context of the invention an uplifted mood may comprise a decreased anxiety level and/or a decreased stress level and/or an increased perceived energy level and/or a more positive emotional state.

Any kind of chocolate or biscuit may be used in accordance with the invention.

As used herein the term chocolate includes milk chocolate, dark chocolate and white chocolate. The term is not intended to be limited to compositions that can legally be described as chocolate in any particular country but extends to any products having the general character of chocolate. The term includes products containing cocoa solids but which contain fats other than cocoa butter. Preferably, the chocolate used in accordance with the invention is milk chocolate, dark chocolate or white chocolate and most preferably the chocolate is milk chocolate.

As used herein the term biscuit means a baked product based on flour and which is generally crisp in texture. The product may optionally be sweetened and may optionally contain fat. Preferably, the biscuit used in accordance with the invention is wafer which may be no- or low-sugar wafer or high-sugar wafer, the latter containing for example at least 10% sugar. Wafer is generally baked as thin sheets with a crisp and delicate texture (see, for example, K F Tiefenbacher in Encyclopaedia of food science, food technology and nutrition, edited by Macrae, Robinson and Sadler, Volume 1, published by Academic Press, 1993, pages 417 to 420).

Most preferably, the chocolate is milk chocolate and the biscuit is wafer. For example, the wafer may be coated with milk chocolate.

In a preferred embodiment the ratio by weight of chocolate to biscuit is from 3:7 to 7:3. Generally the composition will be presented in the form of individual portions for consumption by an individual subject. The individual portions of chocolate and biscuit to be consumed are preferably 'snack' size portions. Each portion may preferably contain from 70 to 180 calories, and may comprise from 0 to 4 g protein, from 5 to 30 g carbohydrate and from 1 to 15 g fat. More preferably each portion contains 100 to 150 calories, and may comprise from 1 to 3 g protein, from 10 to 20 g carbohydrate and from 3 to 10 g fat. Most preferably each individual portion to be consumed contains about 125 calories, and may comprise about 2 g protein, about 15 g carbohydrate and from about 6 g fat.

Compositions comprising chocolate and biscuit can be made by methods well known in the art such as by enrobing the biscuit with molten chocolate or by moulding chocolate around a biscuit centre. In this connection, reference can be made to Biscuits, Cookies and Crackers, Volume 3, Composite Products, by Almond, Gordon, Reardon and Wade, published by Elsevier Applied Science, 1991, chapter 6, Chocolate Covered Products.

A portion of the combination of chocolate and biscuit is usually consumed as a snack in between meals or at mealtimes. Most preferably it is consumed as a snack between mealtimes, and usually alone or with a drink. One or two or more portions of the chocolate and biscuit combination may be consumed daily and most preferably one portion is consumed daily.

The method and composition according to the invention may be particularly suitable for the treatment of a high trait anxiety individual, for example for the treatment of anxiety and/or stress in such an individual. A high trait anxiety individual is an individual who has been classified as being in the high trait anxiety sub-group when individuals are divided into high and low sub-groups by the evaluation of dispositional stress according to a standard trait anxiety scale such as that described in more detail below.

The invention is based on the following experimental work.

### Experimental design and recruitment of volunteers

This study was conducted in accordance with the ethical principles of Good Clinical Practice and the Declaration of Helsinki. The protocol was approved by the Medical Ethics Committee METOPP (Medisch-Ethische Toetsing Onderzoek Patiënten en Proefpersonen / medical ethics review of research with patients and test subjects) on 03 April 2006. All subjects enrolled in the study gave written informed consent. The study was designed as a randomized (by age, gender, trait anxiety), parallel, open study. In total, 157 healthy male and female candidates were selected as potential candidates from the volunteers' database of TNO Quality of Life, Zeist (The Netherlands). The volunteers participation was decided upon determination of health status, lifestyle and eating habits, chocolate eating habits, medical history, age (from 18 to 35 years), body mass index (BMI, from 18 to 25 Kg.m-2), blood pressure, heart rate, as well as blood clinical analyses including haematology, lipidemia (triglycerides, total-cholesterol, high density- and low density-cholesterol) and glycaemia. The exclusion criteria included psychiatric disorders, metabolic or endocrine diseases, gastro-intestinal and eating behaviour disorders, smoking, use of medication that may influence appetite and/or sensory functioning, pregnancy, reported slimming or medically prescribed diet, reported unexplained weight loss or gain in the month prior to the screening, alcohol consumption superior to 21 and 28 units per week for females and males, respectively. A total of 90 subjects were finally enrolled in the study.

### Clinical trial

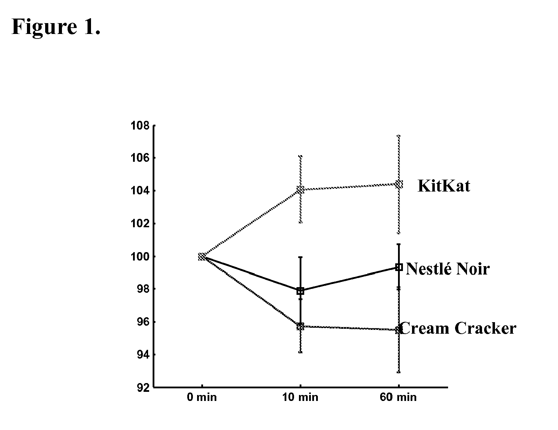

The intervention period lasted two weeks with a daily intake of chocolate and non-chocolate containing snacks. Participants were asked to avoid consumption of chocolate or chocolate containing products during an eight-day run-in period. The study was open for all treatments. Three test days (Days 01, 08, 15) were organized to collect data on the effects of chocolate and non-chocolate snacks on mood-uplifting parameters anxiety state, energy state, emotional state and cognition state (Figure 1). The tested products were wafer fingers with milk chocolate (KitKat mini®, Nestlé Deutschland AS, 60523 Frankfurt am Main, Germany), dark chocolate (Cailler of Switzerland Noir Intense®, Nestlé Suisse SA, Switzerland), and a snack composed of crackers spread with cheese (Jacob's Cream crackers®, The Jacob's bakery Limited, UK). The product intake was calculated to provide similar amount of calories between treatments (Table 1). Therefore, the amount of 25g of KitKat, 20g of dark chocolate and 2 crackers with 15g of cheese were given to participants for each consumption moment. These amounts were considered to be 'snack portions'. The study substances had to be consumed within a time period of 5 minutes. The tested products were given to the participants in their commercial form including original packaging and reported composition. On days 01, 08, and 15 (Table la), tested products were given as a mid-morning snack at TNO at about 10.00 h with a glass of water. The study substances had to be consumed within 5 minutes. From day 02 to day 07, and from day 09 to day 14, study substances were consumed at home or at work daily as a mid-morning and a mid-afternoon snack.

**Table 1: Composition of the treatment products**

| **Composition** | **KitKat** | | **Noir Intense** | **Cream cracker with cheese spread (48+)** | | |
|---|---|---|---|---|---|---|
| | Per 1.5 bar (3 fingers) (=25 g) | 100 g | Per 20 g | 100 g | Per 2 crackers + 15 g cheese spread | 100 g (cracker only) |
| Energy (kcal) | 127.5 | 509 | 114 | 567 | 111 | 438 |
| Protein (g) | 1.7 | 6.7 | 1.8 | 9 | 4.2 | 10.2 |
| Carbohydrate (g) | 15.8 | 62.6 | 5.4 | 27 | 10.9 | 66.7 |
| Fat (g) | 6.5 | 25.6 | 9.4 | 47 | 4.4 | 14.5 |

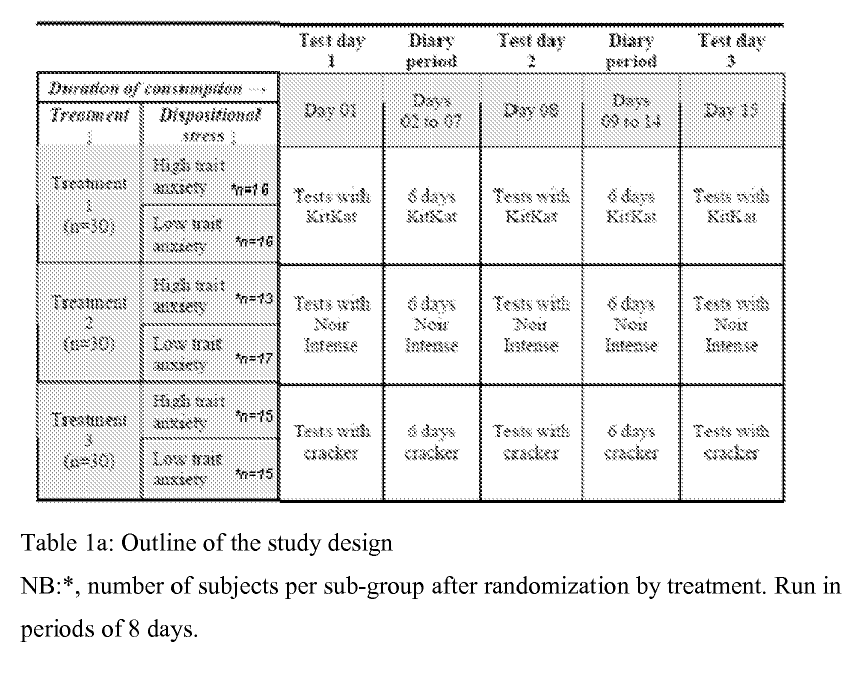

In each treatment group, participants were divided up into either high or low stress sub-group according to the evaluation of their dispositional stress as assessed by scoring on the trait anxiety scale of the State-Trait Anxiety Inventory (STAI) test (Speilberger, Gorsuch & Lushene, 1969). The STAI is the definitive instrument for measuring anxiety in adults which clearly differentiates between the temporary condition of "state anxiety" and the more general and long-standing quality of "trait anxiety." The essential qualities evaluated by the state-anxiety scale are feelings of apprehension, tension, nervousness, and worry. The trait anxiety scale provides an indication of stress, depression and neurosis. Respondents indicated on a 4-point scale to what degree they are experiencing a particular state related to stress and anxiety. For each participant, STAI was applied to determine "trait anxiety" and "state anxiety" that were measured at the beginning of the study and during the treatment period, respectively. In the STAI test, scores from 70 to 78, and from 42 to 64, described low and high trait anxiety subjects, respectively. The study included 28 males and 62 females who were equally distributed between the two trait anxiety conditions as assessed with a Chi-square test (χ²(1)=1.50, p<0.22). Subjects were statistically allocated to an entry number and randomly distributed by treatment considering age, gender, and STAI scores (Table 1a).

On each test day, anxiety, energy and emotional states were measured using subjective reports to questionnaires before (T=0 min) and after (T=10, and 60 min) the intake of the tested products. The state anxiety level was assessed using the State-Trait Anxiety Inventory (STAI; Speilberger, Gorsuch & Lushene, 1969) which clearly differentiates between the temporary condition or "state anxiety" and the more general and long-standing quality defined as "trait anxiety." The STAI test provides a scale of feelings such as apprehension, tension, nervousness, and worry. Therefore, STAI provides an indication of stress, depression and neurosis. Respondents indicated on a 4-point scale to what degree they are experiencing a particular state related to stress and anxiety. The perceived energy levels were measured using the scale of experienced load (SEL; Meijman, 1991) to measure the degree to which participants felt sleepy, alert, and interested for instance. Subjects responded to items in which the anchors were represented by opposing energy states. Respondents indicated on a 4-point scale where their present energy state lies relative to the two anchors. Emotional state was measured using a classification validated by Richins (1997) in a 4-point scale from extremely negative to extremely positive emotions. Finally, cognitive tests were performed using a computer equipped with a colour video monitor, joy stick and push-button panel. Cognitive functioning was assessed using selected tests from the Neurobehavioral Evaluation System (NES) which was originally described by Baker and Letz (Baker et al., 1985) and translated for use in Dutch populations (Emmen et al., 1988; Hooisma et al., 1990). The NES is a computerized test battery designed to evaluate various aspects of cognitive functioning, such as attention, memory and learning, motor performance, and perceptual coding (Hooisma et al., 1990). For this purpose the following tests were performed before (T=0 min) and after (T=30 min) the intake of the tested product: (i) Finger Tapping Test (FTT) for psychomotor performance, (ii) Hand-Eye Coordination Test (HECT) for psychomotor performance, (iii) Simple Reaction Time Test (SRTT) for attention, (iv) Symbol-Digit Substitution Test (SDST) for perceptual coding, and (v) Digit Memory Span Test (DMST) for short-term memory.

### Statistical analysis

Statistical analyses were performed using the Clinical Trials System (CTS), an application based on the SAS V8/V9 Software package. Analyses of variance (ANOVAs) per test day and per time point were done to test the significance of the treatment over time within each trait anxiety subgroup. A statistical model combining all sources of variation (time, stress and treatment) was generated to assess treatment effects and interactions with other factors. The model has a hierarchical structure. As a result, effects on a higher level in the design can be statistically evaluated against the right variation. For example treatment effect is tested against between the pooled subject variations within Stress x Treatment combinations. For the fit a mixed model procedure was used (SAS Proc Mixed).

### Results and Discussion

### Trait anxiety and group classification

In total, the study included 28 males and 62 females distributed across the two trait anxiety conditions high trait anxiety (33 females and 11 males) and low trait anxiety (29 females and 17 males). Chi-square tests showed that the observed distribution did not differ significantly from the ideal distribution (χ²(1) = 1.50, p<.22). Consequently, men and women were equally distributed between the two trait anxiety conditions. T-tests showed that these means differed significantly (t(59) = 19.57, p<.001): the high trait anxiety participants were significantly more anxious than the low trait anxiety subjects. We here report scientific evidence on the effect of certain foods on mood uplifting determinants in these two sub-classes. The observations highlighted an overall impact of a chocolate product together with its commercial context, and the effects cannot be assigned to specific chocolate constituents. The results of the psychological evaluation of subject submitted to a daily intake of chocolate show that effects of statistical significance could be only identified in high trait anxiety individuals.

### Analysis of self-reported data

ANOVAs were conducted on the self-report data for each of the three scales separately (state anxiety, perceived energy levels and emotion). The design tested was: Treatment (KitKat, cream cracker, Noir Intense) x Trait anxiety (high, low) x Time of measurement (0 min - the baseline - , 10 minutes after consumption, 60 minutes after consumption) x Test day (1, 8, 15), with repeated measures on the last two factors. If the analysis of variance indicated a treatment effect (p < 0.05), comparisons between treatments means of the parameters were performed using a 2-sided (paired) Student t-test. The null hypothesis was formulated separately for each variable, and treatment effects were analyzed for each variable. Attention was paid to differences between treatments, between trait anxiety groups and treatment/trait anxiety interaction. Moreover, attention was paid to differences between the baseline measurements of the different test days, and to differences between baseline measurements before consumption of the study products and the measurements taken after consumption of the study substances on the different test days.

Prior to the ANOVAs, the scale reliability was assessed for each of the three scales. Unreliable items or items with little discriminative value were removed from the scales and not used in the definitive analyses. This was done in three steps. First, the variance of the responses was examined for response biases such as ceiling effects. This examination was done by subjectively viewing the distributions and removing items in which only one or two response categories were used or in which the distribution was particularly skewed. Second, factor analyses were applied separately for the remaining state anxiety, energy level and emotion items from the three scales. Each analysis resulted in one usable, interpretable factor. In each case the resulting factor was made up of a limited sub-set of items from the total scale. Third, Cronbach's α (coefficient of reliability or consistency) for each sub-scale was calculated. It indicates the extent to which a set of scale items can be treated as measuring a single one-dimensional latent variable. The state anxiety scale had an α <0.85, the energy scale had an α < 0.82 and the emotion scale had an α < 0.81. All α's indicate that the scales are highly reliable. As a result of these tests, in the definitive analyses only the items remaining after the factor analyses were used. These items were averaged separately for state anxiety, energy levels and emotion, thus resulting in three scores for each participant, which were used as the dependent variables for the subsequent ANOVAs.

### Analysis of effects on state anxiety

Our results demonstrated that only daily intake of KitKat improved anxiety, whilst isocaloric food and dark chocolate induced no significant changes over time. A three-way interaction was found for State anxiety between Trait Anxiety, Treatment and Time (F(4,504) = 4.16, p<0.003) as shown in Table 2. Planned comparisons showed that the effect is the result of changes in the levels of state anxiety for the high trait anxiety subjects over time. Paired comparisons were also calculated and only the significant changes were reported. The mean (± standard error of mean, sem) for the state anxiety score per treatment at different time point for high trait anxiety are shown in Fig. 1. The state anxiety scores for all subjects in the low trait anxiety group did not change significantly over time for all treatments (p >0.05).

**Table 2: Mixed Model Analysis of Variance for the state anxiety scores**

| | Source | DF | Type III SS | Mean Square | F Value | Fr > F |
|---|---|---|---|---|---|---|
| * | Treatment | 2 | 3.346955 | 1.673477 | 0.57 | 0.3828 |
| * | Stress | 1 | 32.971555 | 32.971555 | 19.14 | <.0001 |
| * | Stress*Treatment | 2 | 10.101875 | 5.050937 | 2.98 | 0.0588 |
| | entry(Stress*Treatm) | 84 | 144.714234 | 1.722789 | | |
| * | Day | 2 | 0.451722 | 0.225861 | 1.13 | 0.3245 |
| * | Treatment*Day | 4 | 0.293447 | 0.073362 | 0.37 | 0.8312 |
| * | Stress*Gay | 2 | 0.525152 | 0.262576 | 1.32 | 0.2706 |
| * | Stress*Treatment*Day | 4 | 0.531322 | 0.132831 | 0.67 | 0.6162 |
| | entr*Day(Stre*Treat) | 168 | 33.490347 | 0.199347 | | |
| * | Time | 2 | 0.203040 | 0.101520 | 1.39 | 0.2433 |
| * | Treatment*Time | 4 | 0.440164 | 0.11041 | 1.51 | 0.1981 |
| * | Stress*Time | 2 | 0.39S076 | 0.199033 | 2.73 | 0.0661 |
| | | | | | | |
| | | | | | | |
| * | Day*Time | 4 | 0.527330 | 0.181983 | 1.81 | 0.1254 |
| * | Treatment*Day*Time | 8 | 0.718345 | 0.089793 | 1.23 | 0.2781 |
| * | Stress*Day*Time | 4 | 0.385125 | 0.096281 | 1.32 | 0.2610 |
| | Stres*Treat*Day*Time | 8 | 0.872491 | 0.103031 | 1.50 | 0.1557 |
| | Error: MS(EPPOP) | 504 | 36.737843 | 0.072893 | | |

High trait anxiety subjects receiving KitKat on the test days experienced less stress 10 minutes after consumption compared to baseline, as indicated by higher state anxiety scores (Figure 1). This effect is maintained until one hour after consumption (t=0/t=10: p=0.048; t=0/t=60: p=0.048; t=10/t=60: p=1.000). The cream cracker subjects on the other hand experienced more state anxiety after consumption compared to the baseline, as indicated by lower scores. This effect is maintained for at least an hour after consumption (t=0/t=10: p=0.032; t=0/t=60: p=0.020; t=10/t=60: p=0.85). Subjects who consumed Nestle Noir state anxiety score did not change significantly over time (t=0/t=10: p=0.29; t=0/t=60: p=0.75; t=10/t=60: p=0.46).

### Analysis of effects on perceived energy levels

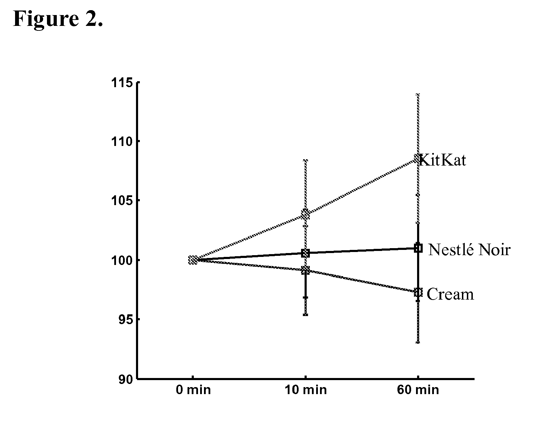

Statistical analysis of psychological data revealed that daily intake of KitKat resulted in an improvement of energy states in high trait anxiety individuals, where as the two other food supplement has no significant impact on perceived energy levels. A three-way interaction between Trait anxiety, Treatment and Time was found for energy as for state anxiety (F(4,504) = 3.30, p<0.01) as shown in Table 3. Planned comparisons showed that the effect is the result of changes in the levels of perceived energy for the high trait anxiety subjects as well as the low trait anxiety subjects over time. The mean (±sem) for the perceived energy score per treatment at different time point for high trait anxiety are shown in Figure 2.

**Table 3: Mixed Model Analysis of Variance for the perceived energy scores**

| Source | | OF | Type III SS | Mean Squars | F Value | Pr > F |
|---|---|---|---|---|---|---|
| * | Treatment | 2 | 0.147116 | 0.073558 | 0.04 | 0.9602 |
| * | Stress | 1 | 46.091662 | 46.091662 | 25.47 | <.0001 |
| * | Stress*Treatment | 2 | 4.165895 | 2.082947 | 1.15 | 0.3212 |
| | entry (Stress*Treatm) | 84 | 152.006688 | 1.809603 | | |
| * | Gay | 2 | 1.017933 | 0.508966 | 1.23 | 0.2942 |
| * | Treatment*Day | 4 | 1.696220 | 0.424055 | 1.03 | 0.3950 |
| * | Stress*Day | 2 | 0.100747 | 0.050374 | 0.12 | 0.8852 |
| * | Stress*Treatment*Day | 4 | 0.473751 | 0.118438 | 0.29 | 0.8862 |
| | entr*Day(Stre*Trest) | 168 | 69.373036 | 0.412935 | | |
| * | Time | 2 | 0.516009 | 0.258005 | 1.32 | 0.2680 |
| * | Treatment*Time | 4 | 0.337785 | 0.084446 | 0.43 | 0.7855 |
| * | Stress*Tme | 2 | 0.427154 | 0.213577 | 1.09 | 0.3360 |
| | | | | | | |
| * | | | | | | |
| * | Day^Time | 4 | 1.711385 | 0.427846 | 2.19 | 0.0691 |
| * | Treatment*Day*Time | 8 | 1.025639 | 0.128330 | 0.66 | 0.7298 |
| * | Stress*Day*Time | 4 | 0.587493 | 0.171873 | 0.88 | 0.4760 |
| | Stres*Treat*Day*Time | 8 | 1.903989 | 0.237999 | 1.22 | 0.2862 |
| | Error: MS(Error) | 504 | 98.493385 | 0.195423 | | |

The KitKat subjects in the high trait anxiety group perceived higher energy levels 60 minutes after consumption compared to baseline (t=0/t=10: p=0.49; t=0/t=60: p=0.033; t=10/t=60: p=0.15) as shown in figure 2. The cream cracker subjects showed no significant changes in perceived energy levels over time (t=0/t=10: p=0.68; t=0/t=60: p=0.28; t=10/t=60: p=0.51). The Nestlé Noir subjects did not report any significant changes over time (t=0/t=10: p=1.0; t=0/t=60: p=0.90; t=10/t=60: p=0.90).

The KitKat subjects in the low trait anxiety group perceived higher energy levels 10 minutes after consumption compared to 60 min after consumption but not compared with baseline (t=0/t=10: p=0.19; t=0/t=60: p=0.50; t=10/t=60: p=0.047). The Cream cracker subjects in the low trait anxiety group perceived higher energy levels 60 minutes after consumption compared to baseline (t=0/t=10: p=0.074; t=0/t=60: p=0.003; t=10/t=60: p=0.26). The black Noir subjects in the low trait anxiety group line does not change significantly over time (t=0/t=10: p=0.61; t=0/t=60: p=0.54; t=10/t=60: p=0.26).

### Analysis of effects on emotion

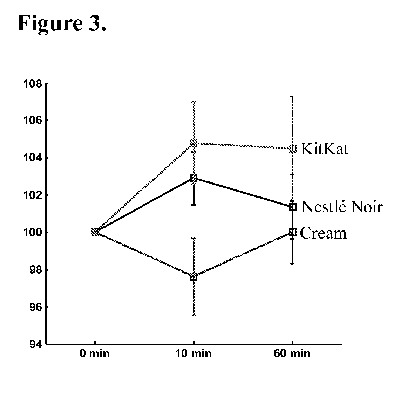

The three-way interaction found for emotion was not significant (F(4,504)=1.71, p<.15) as shown in Table 4. However, intake of Kitkat results in an upward trend towards uplifting emotional state over time, although no statistically significant. The mean (± sem) for the emotion score per treatment at different time point for high trait anxiety subjects are shown in Figure 3. Though, strictly speaking, an interpretation of this interaction is not valid because of the non significant three-way interaction. However, changes can still be interpreted as trends in reported emotion states (Figure 3). Emotion state improvements were reported for KitKat with a positive effect maintained after 1 hour. The Nestlé Noir group reported an upward trend towards improved emotional state after 10 minutes, but not significant. The cream cracker group experienced a decreased emotion state 10 minutes after consumption, this effect disappearing after 1 hour.

**Table 4: Mixed Model Analysis of Variance for the emotion scores**

| | Source | OF | Type III S8 | Mean Square | F Value | Pr > F |
|---|---|---|---|---|---|---|
| * | Treatment | 2 | 3.735556 | 1.859778 | 0.93 | 0.3997 |
| * | Stress | 1 | 33.553470 | 33.559470 | 16.64 | 0.0001 |
| * | Stress*Treatment | 2 | 7.077325 | 3.533663 | 1.75 | 0.1792 |
| | entry(Stress*treatm) | 84 | 169.395515 | 2.016613 | | |
| * | Day | 2 | 0.575626 | 0.287813 | 1.32 | 0.2710 |
| * | Treatment*Day | 4 | 0.195438 | 0.048105 | 0.22 | 0.9244 |
| * | Stress*Day | 2 | 0.381440 | 0.190720 | 0.87 | 0.4200 |
| * | Stress*Treatment*Day | 4 | 0.822960 | 0.205740 | 0.94 | 0.4419 |
| | entr*Day(Stre*Treat) | 168 | 36.743183 | 0.213709 | | |
| * | Time | 2 | 0.784125 | 0.392062 | 5.81 | 0.0032 |
| * | Treatment*Tme | 4 | 0.446519 | 0.111630 | 1.65 | 0.1593 |
| * | Stress*Time | 2 | 0.173481 | 0.086740 | 1.29 | 0.2773 |
| * | Day*Time | 4 | 0.782879 | 0.195718 | 2.90 | 0.0215 |
| * | Treatment*Day*Time | 8 | 0.962329 | 0.120280 | 1.78 | 0.0779 |
| * | Stress*Day*Time | 4 | 0.576354 | 0.144089 | 2.14 | 0.0752 |
| | Stres*Treat*Day*Time | 8 | 0.297844 | 0.037231 | 0.55 | 0.8172 |
| | Error: MS(Error) | 504 | 34.001031 | 0.067462 | | |

The effects of the 3 treatments on anxiety, energy and emotional states are summarized in Table 5 where statistically significant changes are represented by ^{A} and ^{B} for positive and negative effects, respectively. Intake of chocolate has often been associated with improvement of emotional experience such as pleasure, joy and success. Our study showed that only subjects receiving KitKat reported improved anxiety and energy states within the 10 and 60 minutes following consumption. This effect could only be observed in high trait anxiety individuals. So far, only a few investigations reported mental effects induced by chocolate consumption (8, 9), although evidence is sparse and not very solid. Previous work demonstrated that eating a small amount of chocolate reduced negative mood associated with marginal effects on neutral and positive moods (15). Intriguingly, in the present study, perception of mood-related parameters appears to be less dependent on the calorie intake (no effects with the isocaloric substance) than on the composition and the commercial aspect of the consumed product (no effects with dark chocolate). A recent study described how exposure to images of chocolate could affect cravings and negative effects such as guilt, anxiety and depression (16). Such observations suggest that the observed effects could be totally or partially influenced by the commercial image of the used chocolate snack (KitKat). However, it is well documented that improvement of negative mood state, immediately and selectively, is strongly related to the product palatability (15). The observations made in high trait anxiety individuals suggest that the association of a wafer with chocolate could be more efficient than chocolate alone for improving mood within 1 hour after consumption.

**Table 5: Summary of the reported data for perceived anxiety, energy and emotion in high trait anxiety subjects.**

| **Anxiety** | T 10 vs. T0 | T0 vs. T6 | T 10 vs. T60 |
|---|---|---|---|
| KitKat | 0.0477 *^{A} | 0.0477 *^{A} | 1.0000 |
| Nestlé noir | 0.2949 | 0.7532 | 0.4633 |
| Cream cracker | 0.0322 *^{B} | 0.0195 * ^{B} | 0.8453 |
| | | | |

| **Energy** | T 10 vs. T0 | T0 vs. T6 | T10 vs. T60 |
|---|---|---|---|
| KitKat | 0.4889 | 0.0332 * ^{A} | 0.1497 |
| Nestlé noir | 1.0000 | 0.8981 | 0.8981 |
| Cream cracker | 0.6767 | 0.2838 | 0.5123 |
| | | | |

| **Emotion** | T 10 vs. T0 | T0 vs. T6 | T10 vs. T60 |
|---|---|---|---|
| KitKat | 0.0122 * ^{A} | 0.0231 * ^{A} | 0.8137 |
| Nestlé noir | 0.1389 | 0.4330 | 0.4858 |
| Cream cracker | 0.1947 | 0.9353 | 0.2240 |

| | | | |
|---|---|---|---|
| *NB: The statistical significance is expressed at a 95% confidence level (*)*. *^{A}* = *statistically significant changes (positive effect)* *^{B}* = *statistically significant changes (negative effect)* | | | |

### Reference List

1. Michener W, Rozin P, Freeman E et al. The role of low progesterone and tension as triggers of perimenstrual chocolate and sweets craving: some negative experimental evidence. Physiol Behav 1999;67:417-420.
2. Raikkonen K, Matthews KA, Flory JD et al. Effects of optimism, pessimism, and trait anxiety on ambulatory blood pressure and mood during everyday life. J Pers Soc Psychol 1999;76:104-113.
3. Marco CA, Suls J. Daily stress and the trajectory of mood: spillover, response assimilation, contrast, and chronic negative affectivity. J Pers Soc Psychol 1993;64:1053-1063.
4. Davydov DM, Shapiro D, Goldstein IB et al. Moods in everyday situations: effects of combinations of different arousal-related factors. J Psychosom Res 2007;62:321-329.
5. Wood C, Magnello ME. Diurnal changes in perceptions of energy and mood. J R Soc Med 1992;85:191-194.
6. Christensen L. The effect of carbohydrates on affect. Nutrition 1997;13:503-514.
7. Benton D, Donohoe RT. The effects of nutrients on mood. Public Health Nutr 1999;2:403-409.
8. Parker G, Parker I, Brotchie H. Mood state effects of chocolate. J Affect Disord 2006;92:149-159.
9. Macht M, Dettmer D. Everyday mood and emotions after eating a chocolate bar or an apple. Appetite 2006;46:332-336.
10. Smit HJ, Gaffan EA, Rogers PJ. Methylxanthines are the psycho-pharmacologically active constituents of chocolate. Psychopharmacology (Berl) 2004;176:412-419.
11. Drewnowski A, Krahn DD, Demitrack MA et al. Taste responses and preferences for sweet high-fat foods: evidence for opioid involvement. Physiol Behav 1992;51:371-379.
12. Markus CR, Panhuysen G, Jonkman LM et al. Carbohydrate intake improves cognitive performance of stress-prone individuals under controllable laboratory stress. Br J Nutr 1999;82:457-467.
13. Wurtman RJ. Nutrients affecting brain composition and behavior. Integr Psychiatry 1987;5:226-238.
14. Papanikolaou Y, Palmer H, Binns MA et al. Better cognitive performance following a low-glycaemic-index compared with a high-glycaemic-index carbohydrate meal in adults with type 2 diabetes. Diabetologia 2006;49:855-862.
15. Macht M, Mueller J. Immediate effects of chocolate on experimentally induced mood states. Appetite 2007;49:667-674.
16. Fletcher BC, Pine KJ, Woodbridge Z et al. How visual images of chocolate affect the craving and guilt of female dieters. Appetite 2007;48:211-217.

## Claims

1. A method of uplifting the mood of a human subject which comprises administering to a human subject in need thereof a composition which comprises a combination of chocolate and biscuit.

2. The method of claim 1 wherein uplifting the mood of said subject comprises decreased anxiety level and/or decreased stress level and/or increased perceived energy level and/or more positive emotional state.

3. The method of claim 1 or 2 wherein the human subject is a high trait anxiety individual.

4. The method of any preceding claim wherein the chocolate is milk chocolate, dark chocolate or white chocolate, preferably milk chocolate.

5. The method of any preceding claim wherein the biscuit is wafer, preferably wafer containing sugar.

6. The method of any preceding claim wherein the composition comprises chocolate milk chocolate and wafer.

7. The method of claim 6 wherein the composition comprises wafer coated with milk chocolate.

8. The method of any preceding claim wherein the composition comprises chocolate and biscuit in a ratio by weight of from 3:7 to 7:3.

9. The method of any preceding claim wherein the composition is presented as individual portions containing from 70 to 180 calories, and comprising from 0 to 4 g protein, from 5 to 30 g carbohydrate and from 1 to 15 g fat.

10. A composition comprising a combination of chocolate and biscuit for use in a method as defined in any of claims 1 to 9.
